# DEMANDE DE BREVET EUROPEEN

(11) **EP 1 650 165 A1**
(43) Date de publication de la demande: **26.04.2006**
(21) Numéro de dépôt: 05109348.2
(22) Date de dépôt: 07.10.2005
(51) Int. Cl.: C02F 1/02, F24D 17/00

(54) **Dispositif de réchauffage et de pasteurisation en continu d'un fluide**

(30) Priorité: 08.10.2004 FR 0452313
(71) Demandeur: Boiffier, Jean-Jacques, 33390 Mazion (FR)
(72) Inventeur: Boiffier, Jean-Jacques, 33390, Mazion (FR)
(74) Mandataire: Schmit, Christian Norbert Marie

(57) **Abrégé**

L'invention concerne un dispositif de réchauffage et de pasteurisation d'un fluide comprenant un volume délimité par des parois (1) apte à contenir un fluide, ce volume ayant une partie haute (2) et une partie basse (3). Le dispositif comprend une alimentation en fluide (4) à traiter à une température d'entrée Tₑ. Selon l'invention, ce dispositif comprend un échangeur double flux (11) placé en partie basse (3) dudit volume et relié à ladite alimentation (4) à une de ses extrémités. Il comprend un élément de transport (12) du fluide pour amener ledit fluide en partie haute (2) dudit volume, ledit élément de transport (12) étant relié à l'autre extrémité dudit échangeur double flux (11). Il comprend également un échangeur de pasteurisation (13) pour porter la température de ce fluide à une température de pasteurisation Tp, ce fluide à ladite température de pasteurisation Tₚ étant destiné à circuler en partie haute (2) dudit volume pendant une durée tₚ avant que sa température ne soit diminuée par ledit échangeur double flux (11) d'une valeur au moins égale à ΔT pour atteindre une valeur de distribution T_{d}.

## Description

La présente invention concerne un dispositif de réchauffage et de pasteurisation en continu d'un fluide.

Depuis la description de la première épidémie de légionelloses survenue en 1976, de nombreuses études ont permis de mieux connaître les modes de développement et de propagation de la bactérie *Legionella pneumophila* afin de prévenir sa prolifération.
Le réservoir de la bactérie est essentiellement hydrique. Elle prolifère dans les installations d'eau dés que la température est supérieure ou égale à 23°C et inférieure à 50°C. Elle prolifère également lorsque l'eau stagne ou circule mal (points d'usage non utilisés, bras morts, réservoirs,...). La transmission à l'homme s'effectue quant à elle par simple inhalation d'eau contaminée en suspension dans l'air. Les dispositions préventives visent donc prioritairement les installations de bâtiments collectifs ou individuels produisant des aérosols et ceux particulièrement pourvus de douches ou douchettes.
On connaît diverses méthodes de désinfection des réseaux sanitaires. Une première méthode vise à traiter chimiquement ces réseaux par introduction dans les canalisations de produits chimiques essentiellement à base de chlores ou de peroxydes. Néanmoins, ce type de traitement ne peut avoir qu'un caractère ponctuel et n'a donc pas un caractère rémanent. Il nécessite de plus un rinçage important afin que l'eau délivrée en fonctionnement normal par le réseau soit potable.
On préfère donc mettre en oeuvre un traitement thermique continu du réseau afin de limiter le développement des légionelles.
On connaît par le brevet FR 2812815 du demandeur, un procédé et un dispositif de traitement sanitaire simultané d'un fluide et de son réseau. Ce dispositif de traitement comprend notamment un circuit de pasteurisation du fluide à traiter à une température de pasteurisation Tₚ pendant une durée de pasteurisation qui est fonction de cette température. Ce circuit de pasteurisation comprend une Lyre recevant directement le fluide ayant circulé dans un échangeur de pasteurisation. Cette Lyre permet de maintenir le fluide pasteurisé pendant cette durée de pasteurisation tp avant que le fluide pasteurisé ne pénètre dans un échangeur double flux, cet échangeur diminuant la température du fluide pasteurisé d'une valeur Tₚ vers une température de distribution T_{d}. Ce dispositif permet avantageusement d'obtenir un traitement thermique sûr et efficace d'un fluide et de maintenir ce fluide en circulation permanente dans le circuit de distribution sous traitement, tout en assurant le traitement simultané, en continu du réseau.
Cependant les qualités de ce dispositif peuvent encore être améliorées. La Lyre de pasteurisation présente un volume d'encombrement important, en particulier dans le cas de gros débits à maintenir en température de pasteurisation. Pour donner un exemple, un débit de 10m³/h nécessite de l'ordre de 30 m de tuyauterie d'un diamètre nominal 100 pour engendrer une durée de circulation adaptée. De plus, ce dispositif fonctionne en régime de production de type quasi-instantané car la Lyre ne permet qu'une durée limitée de maintien en température de pasteurisation. Une température de pasteurisation élevée, supérieure ou égale à 70°C, est nécessaire pour assurer l'éradication totale des micro-organismes sur cette durée limitée. La source de chaleur du type échangeur primaire (alimentation en fluide calorifique et tuyauterie) ou chaudière assurant l'apport en chaleur à l'échangeur de pasteurisation doit donc toujours fonctionner à des températures élevées de l'ordre de 85-90°C occasionnant des coûts de consommation d'énergie non négligeables.
Par ailleurs, les dispositifs de l'art antérieur sont encombrants et nécessitent de larges volumes à leur installation ce qui rend difficile leur mise en oeuvre dans de petites structures. De plus, les différentes parties de ces dispositifs rayonnent occasionnant des pertes d'énergie parfois importantes.

L'objectif de la présente invention est donc de proposer un dispositif de réchauffage et de pasteurisation en continu d'un fluide simple dans sa conception et dans son mode opératoire, économique, compact et permettant de disposer d'un volume dont la contenance est telle que le temps de séjour du fluide à la température de pasteurisation est largement augmenté autorisant ainsi des températures de pasteurisation moindres tout en présentant très peu de pertes énergétiques. Une telle limitation des pertes d'énergie est due à la compacité du dispositif, i.e. à la limitation des tuyauteries mises en oeuvre pour le transport du fluide par rapport aux dispositifs de l'art antérieur. Le dispositif de l'invention présente à la fois une faible emprise au sol et une sensible amélioration de la compacité en hauteur.

Ce dispositif permet également une amélioration de l'échange interne d'énergie entre le fluide pasteurisé et l'échangeur double flux. Il est d'une installation rapide et particulièrement simple pour un opérateur.

A cet effet, l'invention concerne un dispositif de pasteurisation d'un fluide comprenant un volume délimité par des parois apte à contenir un fluide, ce volume ayant une partie haute et une partie basse. Le dispositif comprend une alimentation en fluide à traiter à une température d'entrée Tₑ.
Selon l'invention, ce dispositif comprend :
- un échangeur double flux placé en partie basse de ce volume et relié à une de ses extrémités à ladite alimentation, cet échangeur double flux étant destiné à augmenter la température d'un fluide entrant d'une valeur ΔT donnée pour atteindre une valeur Tₑ+ΔT,
- un élément de transport du fluide pour amener ce fluide en partie haute du volume, cet élément de transport étant relié à l'autre extrémité de l'échangeur double flux,
- un échangeur de pasteurisation pour porter la température de ce fluide d'une température Tₑ+ ΔT vers une température de pasteurisation Tₚ,
- ce fluide à la température de pasteurisation Tₚ étant destiné à circuler en partie haute du volume pendant une durée tp avant que sa température ne soit diminuée par l'échangeur double flux d'une valeur au moins égale à ΔT pour atteindre une valeur de distribution T_{d}.
On entend par "Fluide", toute substance capable de couler facilement et susceptible de contenir des micro-organismes dont on cherche à obtenir l'éradication par pasteurisation. Dans le but d'exposer la présente invention à travers des modes de réalisation préférés, on s'intéressera tout particulièrement dans la description qui va suivre à la stérilisation de l'eau dans le cadre des réseaux d'eau chaude sanitaire parce que l'application à ce domaine est un problème important, ceci pour de nombreux lieux et plus particulièrement pour ceux qui reçoivent du public. Néanmoins, l'invention ne saurait être limitée à ces seuls modes de réalisation qui ne sont donnés qu'à titre illustratif. L'invention peut en effet adresser d'autres problèmes tels que la pasteurisation et/ou la stérilisation d'un fluide alimentaire pour détruire les micro-organismes pathogènes avant conditionnement.
Dans différents modes de réalisation particuliers du dispositif de pasteurisation d'un fluide, chacun ayant ses avantages particuliers et susceptibles de nombreuses combinaisons techniques possibles:
- l'échangeur de pasteurisation est placé dans la partie haute du volume,
- l'échangeur de pasteurisation comprend au moins une épingle électrique,
- l'élément de transport du fluide comprend l'échangeur de pasteurisation,
- l'échangeur de pasteurisation est relié à un circuit primaire,
- le dispositif comprend des moyens pour optimiser le temps de séjour du fluide dans la partie haute du volume de sorte à maintenir ce fluide pendant une durée tp dans cette partie haute,
- les moyens pour optimiser le temps de séjour du fluide comportent une canule d'injection reliée à l'élément de transport du fluide de sorte à introduire le fluide dans la partie haute du volume sensiblement tangentiellement à la paroi interne du dispositif,
- les moyens pour optimiser le temps de séjour du fluide comprennent au moins un élément brise-jet pour diffuser le fluide en partie haute dudit volume à faible vitesse et sans turbulence,
- l'échangeur double flux est un échangeur serpentin à au moins un enroulement,
- le dispositif comprend un piquage pour choc thermique curatif ou by-pass thermique destiné à être relié à un circuit de distribution du fluide,
- le dispositif comprend un piquage d'utilité placé dans la partie basse du volume de manière à ce que le fluide soutiré soit à une température inférieure à Tₚ mais supérieure à Tₚ-ΔT,
- le dispositif comprend un piquage de vidange pour chasse rapide placé en bas de la partie basse du volume,
- le dispositif comprend des moyens de contrôle et de sécurité de la température du fluide placés en partie haute du volume,
- le circuit primaire comprend une source en fluide caloporteur et des moyens d'actionnement de cette source, les moyens d'actionnement étant commandés par les moyens de contrôle de la température du fluide.

L'invention concerne également un réseau sanitaire pour l'alimentation en fluide de points de distribution. Selon l'invention, ce réseau comprend un dispositif de pasteurisation d'un fluide tel que décrit précédemment.
Un objectif de la présente invention est de réaliser une stérilisation du réseau de distribution associé en circulation permanente par traitement thermique.
De préférence, le réseau comprend un circuit de bouclage du fluide alimentant en continu le dispositif de pasteurisation.
Le dispositif de pasteurisation comprend un ou plusieurs circulateurs ou pompes.

L'invention sera décrite plus en détail en référence aux dessins annexés dans lesquels:
- la figure 1 est une représentation schématique du dispositif de pasteurisation d'un fluide selon un premier mode de réalisation de l'invention;
- la figure 2 est une représentation schématique du dispositif de pasteurisation d'un fluide selon un deuxième mode de réalisation de l'invention;
- la figure 3 est une représentation schématique des moyens pour optimiser le temps de séjour du fluide dans ladite partie haute du volume selon un premier mode de réalisation de l'invention;
- la figure 4 est une représentation schématique des moyens pour optimiser le temps de séjour du fluide dans ladite partie haute du volume selon un deuxième mode de réalisation de l'invention;
- la figure 5 est une représentation schématique d'un réseau sanitaire pour l'alimentation en fluide de points de distribution selon un mode de réalisation particulier de l'invention;

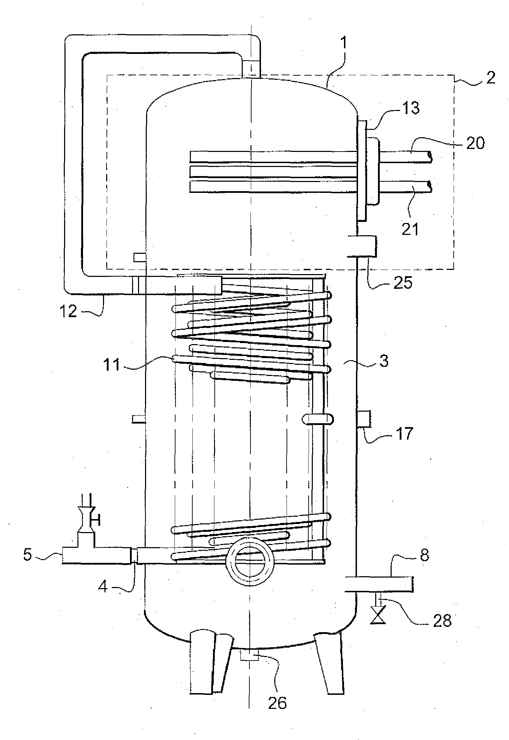

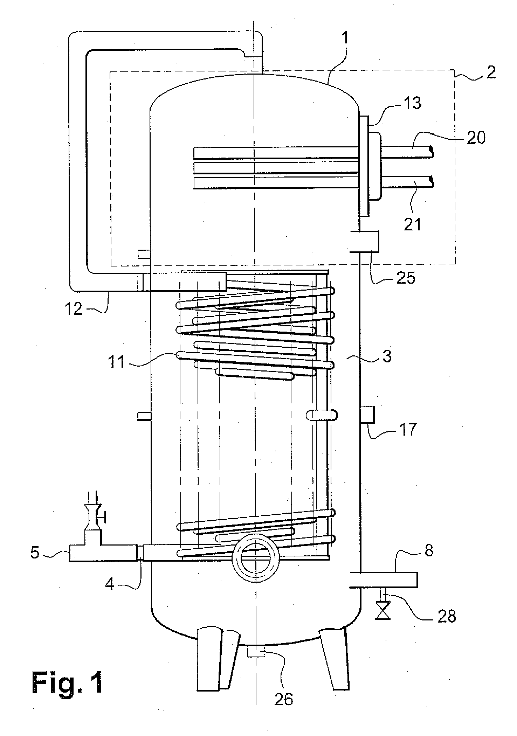

La Figure 1 montre un dispositif de pasteurisation d'un fluide selon un premier mode de réalisation de l'invention. Le dispositif comprend un volume délimité par des parois 1, ce volume étant apte à contenir un fluide. Ce volume est variable. Il comprend une partie haute 2 et une partie basse 3.

Les parois 1 sont par exemple en acier inoxydable ou en acier carbone revêtu lisse de manière à empêcher le calcaire d'adhérer sur les surfaces en contact avec le fluide pasteurisé. Ces parois 1 peuvent de plus comporter une jaquette calorifugée comprenant un isolant fibreux (laine de verre, laine de roche, ...) ou un isolant pulvérulent (silicate de calcium,...) pour limiter les pertes d'énergie.

Le dispositif comprend une alimentation en fluide 4 à traiter à une température d'entrée Tₑ. Cette alimentation en fluide 4 est par exemple reliée à un circuit d'alimentation 5 en eau froide sous pression à partir du réseau public. Cette eau froide dans ce cas est destinée à fabriquer de l'eau chaude sanitaire et est habituellement à une température de l'ordre de 10 à 15°C. Elle peut néanmoins être à des températures plus élevées, l'eau étant par exemple pré-chauffée.

Préférentiellement, cette alimentation 4 est reliée par l'intermédiaire d'une vanne de réglage 6 à un circuit de bouclage 7 en continu du fluide. Ce circuit de bouclage 7 est alors relié par son autre extrémité à un circuit de distribution de l'eau chaude sortant du dispositif de pasteurisation à une température de distribution Tₚ par l'intermédiaire d'un piquage de distribution 8 placée dans la région inférieure de la partie basse 3 dudit volume. Ce circuit de bouclage 7 permet de traiter en continu le fluide pasteurisé circulant dans le circuit de distribution pour réchauffer ce fluide. Il comprend des moyens de mise en circulation dudit fluide. Ces moyens comprennent au moins une pompe 9 et l'instrumentation 10 (manomètres, soupapes de sécurité,...) nécessaire au contrôle de la circulation d'un fluide sous pression, laquelle est connue de l'homme de métier.

La température d'entrée Tₑ du fluide à traiter est déterminée par le mélange de l'eau froide ou préchauffée et de l'eau de bouclage. Elle est par exemple comprise entre 23°C et 50°C dans l'exemple particulier de l'eau chaude sanitaire selon que l'eau de retour issue du circuit de bouclage 7 est mélangée ou non avec l'eau froide. Ces conditions peuvent bien évidement varier en fonction des utilisations.

Cette eau à la température d'entrée Tₑ est ensuite envoyée dans un échangeur double flux 11 placé en partie basse du volume. Cet échangeur double flux 11 augmente la température du fluide entrant d'une valeur ΔT donnée pour atteindre une valeur Tₑ+ΔT. Dans un mode de réalisation préféré, cette valeur ΔT est égale ou supérieure à 11°C. L'échangeur double flux 11 comprend un échangeur serpentin à triple enroulement.

Cet échangeur double flux 11 est relié à son autre extrémité à un élément de transport du fluide 12. Cet élément de transport 12 comprend par exemple un tuyau de section adaptée au débit du fluide pour amener ce fluide en partie haute 2 du volume. Lorsque ce tuyau est externe aux parois 1 délimitant la partie haute 2 du volume, il est avantageusement calorifugé pour limiter les pertes d'énergie.

Le dispositif comprend également un échangeur de pasteurisation 13 pour porter la température de l'eau d'une température Tₑ+ΔT vers une température de pasteurisation Tₚ. Cet échangeur de pasteurisation 13 est placé en partie haute 2 du volume. Il comprend alors soit un ou plusieurs échangeurs tubulaires noyés, raccordés sur un circuit primaire de fluide caloporteur sous pression, soit au moins une épingle électrique noyée, soit un brûleur à combustible et son échangeur de gaz brûlés. La température de pasteurisation est toujours supérieure à la température d'éradication des micro-organismes. Avantageusement, elle est strictement comprise entre 65°C et 75°C dans le cas de l'eau chaude sanitaire et pour le traitement de la légionelle.

Le fluide à la température de pasteurisation Tₚ circule en partie haute 2 du volume pendant une durée tp nécessaire à l'élimination des micro-organismes, cette durée étant fonction de la température de pasteurisation et de la nature des micro-organismes. Ce maintien en température de pasteurisation peut être obtenu par le dimensionnement de la partie haute 2. La partie haute 2 du volume présente alors une hauteur telle que le fluide arrivé en partie haute 2 du volume et se déplaçant vers sa partie basse 3 met un temps tp pour traverser cette partie haute. A titre d'exemple de mise en oeuvre, pour un dispositif comprenant un récipient de diamètre interne de 650 mm et ayant un volume de 500 L, et pour un élément de transport ayant un diamètre de 50 mm et pour une vitesse du fluide dans cet élément de 0,65 ms-¹, la hauteur h de la partie haute 2 est de 350 mm afin de maintenir l'eau pendant au moins deux minutes à une température Tₚ strictement comprise entre 65 et 75°C.

L'eau pasteurisée ayant quitté la partie haute 2 du volume, est refroidie graduellement par contact avec la paroi externe de l'échangeur double flux 11. La température de l'eau se déplaçant de haut en bas dans la partie basse 3 du volume diminue d'une valeur au moins égale à ΔT pour atteindre une valeur de distribution T_{d} = Tₚ - ΔT. Dans un mode de réalisation préféré, cette valeur ΔT est égale ou supérieure à 11°C.

L'eau à la température de distribution T_{d} est ensuite envoyée dans un circuit de distribution 14 par l'intermédiaire d'un piquage de distribution 8 placé en dans une région inférieure de la partie basse 3 du volume. Ce circuit de distribution 14 distribue le fluide pasteurisé vers des postes de distribution 15, ces postes 15 étant par exemple reliés également à un circuit d'eau froide 16. La température de distribution est supérieure à la température d'éradication des micro-organismes; c'est-à-dire supérieure à 55°C, à la sortie du dispositif.

Un piquage d'utilité 17 de diamètre approprié est placé sur le dispositif dans une région intermédiaire de la partie basse 3 du volume de manière à créer un puisage spécifique tel que la température de l'eau chaude stérilisée, déjà légèrement refroidie par son passage sur l'échangeur double flux, se situe à une température d'utilisation comprise entre 60 et 65°C en fonctionnement normal. Ce piquage 17 est créé pour alimenter à cette température d'utilisation des points de distribution 18 ayant des besoins spécifiques tels que cuisine, machines à laver,....

Dans un mode de réalisation préférentiel, le circuit de distribution 7 comporte une vanne de réglage trois voies à laquelle est reliée d'une part un circuit de bouclage 7 et d'autre part une dérivation reliée à ce piquage d'utilité 17. Ainsi, du fluide pasteurisé ayant déjà été légèrement refroidi par l'échangeur double flux 11 est introduit dans le circuit de distribution 14 pour faire varier à la demande la température de distribution du fluide pasteurisé. Dans le cas de l'eau chaude sanitaire, cette température de distribution peut ainsi être variée entre 55 et 60°C, par exemple.

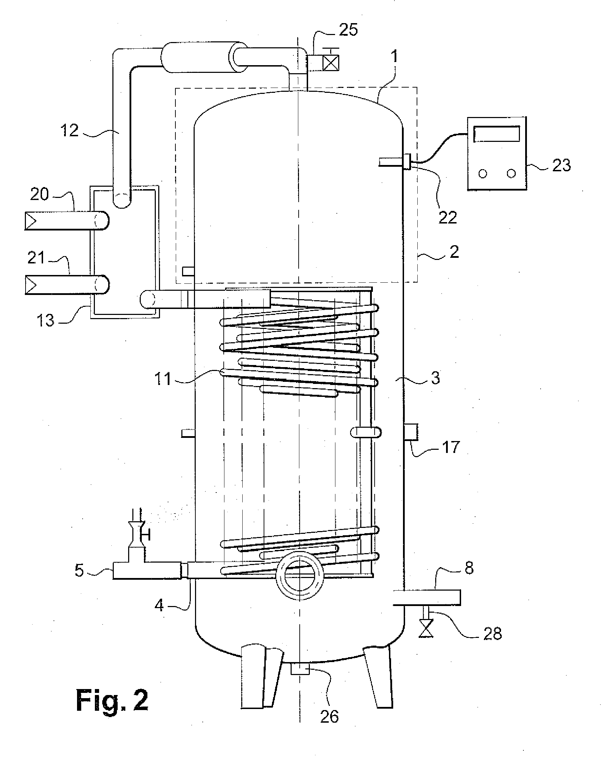

La Figure 2 montre un autre mode de réalisation du dispositif de pasteurisation d'un fluide selon l'invention. Les éléments de la Figure 2 ayant les mêmes références que sur la Figure 1 désignent les mêmes objets. Dans ce mode de réalisation, l'élément de transport 12 comprend l'échangeur de pasteurisation 13, ce dernier étant alors externe au volume. Cet échangeur 13 est relié à une de ses extrémités à l'échangeur double flux 11 pour recevoir le fluide à une température Tₑ+ΔT et à son autre extrémité à un conduit avantageusement calorifugé de l'élément de transport 12 qui transporte le fluide pasteurisé en partie haute 2 du volume. Cet échangeur de pasteurisation 13 est par exemple un échangeur à plaques, brasées ou non, relié à un circuit primaire 19 comprenant une source 20 en fluide caloporteur avec un retour 21 pour le fluide caloporteur ayant apporté un gain de calories au fluide entrant dans l'échangeur 13 à une température Tₑ+ΔT. L'échangeur de pasteurisation 13 peut également être un brûleur à combustible, par exemple, un générateur à gaz ou des résistances électriques.

Le dispositif comprend avantageusement des moyens de contrôle et de sécurité de la température du fluide tels qu'une sonde de mesure 22 en température de la partie haute du volume, reliée à un thermostat 23 de régulation. Ce thermostat de régulation 23 commande des moyens d'actionnement de cette source en fluide caloporteur, par exemple, une unité de pompage 24. Il comprend en outre une sonde de sécurité permettant de couper les moyens d'actionnement de la source en fluide caloporteur en cas de dépassement d'une température de sécurité prédéfinie, 75°C par exemple. Le circuit primaire 19 peut comprendre de plus une électrovanne à trois voies pour régler le débit en fluide caloporteur, le thermostat 23 étant alors un thermostat à double contact dont un contact est un contact de sécurité haute température plaçant au-delà d'un seuil de température prédéfinie, par exemple 75°C, automatiquement l'électrovanne à trois voies en position fermée sur la voie venant de la source en fluide caloporteur. Alternativement, ces moyens de contrôle de la température du fluide pasteurisé peuvent comprendre une unité de régulation électronique réglant automatiquement la température de pasteurisation à partir d'une électrovanne à trois voies reliée au circuit primaire. Un dispositif optique, par exemple une caméra CCD reliée à des dispositifs de visualisation du type écran et d'alerte permet une surveillance à distance en continu du bon fonctionnement thermique du dispositif et du réseau qu'il alimente.

Le dispositif comporte avantageusement un piquage 25 pour choc thermique curatif, ou by-pass thermique, lequel est de diamètre approprié et placé en aval de l'échangeur de pasteurisation soit sur l'élément de transport lorsque l'échangeur de pasteurisation est externe, soit sur les parois délimitant la partie haute du volume lorsque ce dernier est intégré au volume. Ce piquage 25 est relié par l'intermédiaire d'une vanne et d'une dérivation au circuit de distribution 14 du fluide. Ce piquage 25 permet de désinfecter les canalisations du circuit de distribution.

Le dispositif peut comporter un piquage de prélèvement 28 de faible diamètre armé d'une vanne, placé sur la tangente inférieure du piquage de distribution 8 en vue de faciliter des prélèvements d'eau pour tests physicochimiques et bactériologiques.

Le dispositif comporte par ailleurs un piquage de vidange 26 pour chasse rapide par exemple en vue d'un désembouage ou d'une vidange du dispositif pour une intervention. Ce piquage 26 est placé de préférence à l'extrémité de la région inférieure de la partie basse du volume.

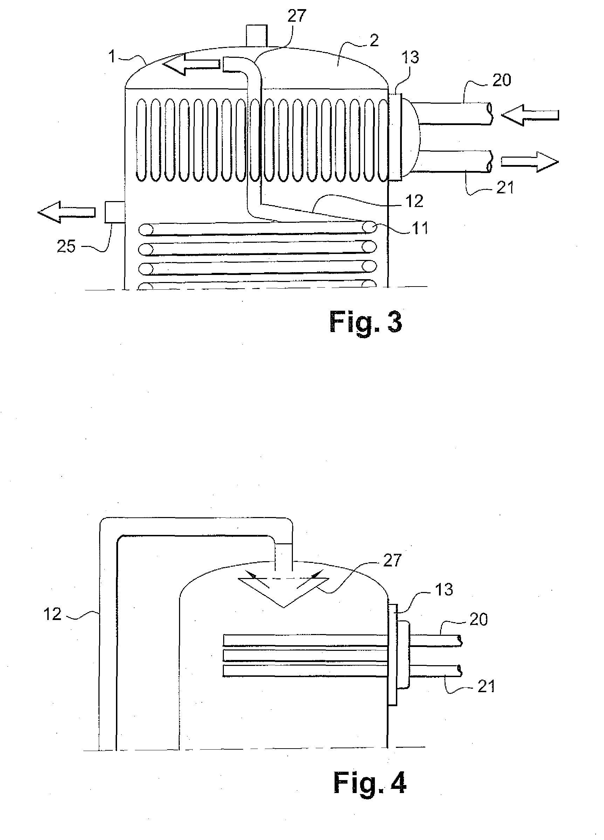

Les figures 3 et 4 sont des représentations schématiques des moyens pour optimiser le temps de séjour du fluide 27 dans ladite partie haute 2 du volume. Sur la figure 3, ces moyens pour optimiser le temps de séjour du fluide 27 comportent une canule d'injection orientée qui est reliée à l'élément de transport du fluide 12 de sorte à introduire le fluide dans la partie haute 2 du volume sensiblement tangentiellement à la paroi 1 interne de ce volume. L'élément de transport 12 est interne audit volume et est relié à son extrémité amenant le fluide en partie haute 2 du volume à une canule d'injection orientée 27. Ce mode de réalisation permet d'améliorer encore la compacité du dispositif de pasteurisation.
Sur la figure 4, ces moyens pour optimiser le temps de séjour du fluide 27 comprennent un élément brise-jet pour diffuser le fluide en partie haute du volume à faible vitesse et sans turbulence. Cet élément permet par exemple d'éviter que le fluide sous pression n'emprunte des chemins préférentiels et se retrouve en partie basse du volume en un temps inférieur à la durée tp nécessaire à l'élimination des micro-organismes. Cet élément brise-jet comporte dans ce mode de réalisation particulier une coupelle inversée, le fluide amené par l'élément de transport s'écoulant le long de sa surface externe concave.

Ces moyens pour optimiser le temps de séjour du fluide 27 permettent de maintenir le fluide plus longtemps dans la partie haute 2 du volume ce qui permet de réduire par exemple la hauteur physique de cette partie haute 2 et d'éviter un passage direct et vertical du fluide ce qui aurait pour conséquence de limiter le temps de pasteurisation.

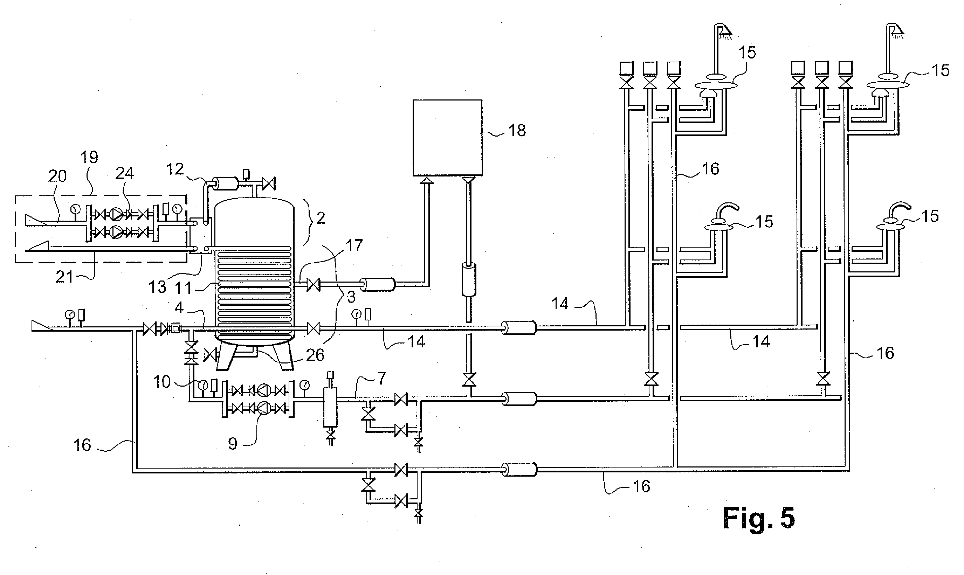

La Figure 5 montre un réseau sanitaire pour l'alimentation en fluide de postes de distribution 15 dans un mode de réalisation particulier de l'invention.
L'invention ne saurait être limitée à la description qui précède et qui a été donnée dans le cas particulier de la stérilisation de l'eau chaude sanitaire. Elle est par ailleurs susceptible de modifications avec l'évolution des technologies. Des substitutions et/ou des modifications dans la structure générale et dans les détails du présent dispositif de pasteurisation et du présent réseau peuvent être réalisées par un homme du métier sans s'écarter de l'esprit de la présente invention. Ainsi, le dispositif peut être adapté pour traiter d'autres fluides tels que des effluents, des fluides chargés ou des produits liquides alimentaires. Le traitement de certains effluents, par exemple des rejets d'usine, nécessite ainsi des températures de pasteurisation de l'ordre de 120 à 150°C. Le circuit primaire est alors un circuit primaire à vapeur et la source primaire est une chaudière produisant de la vapeur à haute pression.

## Revendications

1. Dispositif de réchauffage et de pasteurisation d'un fluide comprenant un volume délimité par des parois (1) apte à contenir un fluide, ledit volume ayant une partie haute (2) et une partie basse (3), ledit dispositif comprenant une alimentation en fluide (4) à traiter à une température d'entrée Tₑ, **caractérisé en ce qu'**il comprend:
- un échangeur double flux (11) placé en partie basse (3) dudit volume et relié à une de ses extrémités à ladite alimentation (4), ledit échangeur double flux étant destiné à augmenter la température d'un fluide entrant d'une valeur ΔT donnée pour atteindre une valeur Tₑ+ΔT,
- un élément de transport (12) du fluide pour amener ledit fluide en partie haute (2) dudit volume, ledit élément de transport (12) étant relié à l'autre extrémité dudit échangeur double flux (11),
- un échangeur de pasteurisation (13) pour porter la température de ce fluide d'une température Tₑ+ ΔT vers une température de pasteurisation Tₚ,
- ce fluide à la température de pasteurisation Tₚ étant destiné à circuler en partie haute (2) du volume pendant une durée tp avant que sa température ne soit diminuée par l'échangeur double flux (11) d'une valeur au moins égale à ΔT pour atteindre une valeur de distribution Td.

2. Dispositif selon la revendication 1, **caractérisé en ce que** ledit échangeur de pasteurisation (13) est placé dans la partie haute (2) dudit volume.

3. Dispositif selon la revendication 2, **caractérisé en ce que** ledit échangeur de pasteurisation (13) comprend au moins une épingle électrique.

4. Dispositif selon la revendication 1, **caractérisé en ce que** ledit élément de transport du fluide (12) comprend ledit échangeur de pasteurisation (13).

5. Dispositif selon la revendication 2 ou 4, **caractérisé en ce que** ledit échangeur de pasteurisation (13) est relié à un circuit primaire.

6. Dispositif selon l'une quelconque des revendications 1 à 5, **caractérisé en ce que** ledit dispositif comprend des moyens pour optimiser le temps de séjour (27) dudit fluide dans ladite partie haute (2) de sorte à maintenir ledit fluide pendant une durée tp dans la partie haute (2) dudit volume.

7. Dispositif selon la revendication 6, **caractérisé en ce que** lesdits moyens pour optimiser le temps de séjour dudit fluide (27) comportent une canule d'injection reliée audit élément de transport du fluide de sorte à introduire le fluide dans ladite partie haute (2) du volume sensiblement tangentiellement à la paroi interne (1) dudit dispositif.

8. Dispositif selon la revendication 6, **caractérisé en ce que** lesdits moyens pour optimiser le temps de séjour dudit fluide (27) comprennent au moins un élément brise-jet pour diffuser ledit fluide en partie haute dudit volume à faible vitesse et sans turbulence.

9. Dispositif selon l'une quelconque des revendications 1 à 8, **caractérisé en ce que** ledit échangeur double flux (11) est un échangeur serpentin à au moins un enroulement.

10. Dispositif selon l'une quelconque des revendications 1 à 9, **caractérisé en ce que** ledit dispositif comprend un piquage d'utilité (17) placé dans la partie basse dudit volume de manière à ce que le fluide soutiré soit à une température inférieure à Tₚ mais supérieure à Tₚ-ΔT.

11. Dispositif selon l'une quelconque des revendications 1 à 10, **caractérisé en ce que** ledit dispositif comprend des moyens de contrôle et de sécurité (22, 23) de la température du fluide placés en partie haute (2) dudit volume.

12. Dispositif selon les revendications 5 et 11, **caractérisé en ce que** ledit circuit primaire comprend une source en fluide caloporteur et des moyens d'actionnement de ladite source, lesdits moyens d'actionnement étant commandés par lesdits moyens de contrôle et de sécurité (22, 23) de la température du fluide.

13. Réseau sanitaire pour l'alimentation en fluide de points de distribution, **caractérisé en ce qu'**il comprend un dispositif de réchauffage et de pasteurisation d'un fluide selon l'une quelconque des revendication 1 à 12.

14. Réseau sanitaire selon la revendication 13, **caractérisé en ce qu'**il comprend un circuit de bouclage continu (7) du fluide, ledit circuit de bouclage étant relié à ladite alimentation en fluide (4) à traiter.
